# EUROPÄISCHE PATENTSCHRIFT

(11) **EP 1 583 731 B1**
(45) Veröffentlichungstag und Bekanntmachung des Hinweises auf die Patenterteilung: **14.05.2008**
(21) Anmeldenummer: 03789416.9
(22) Anmeldetag: 24.12.2003
(51) Int. Cl.: C07C 51/44, C07C 67/54, C07C 45/82, B01D 3/16

(54) **VERFAHREN DER REKTIFIKATIVEN AUFTRENNUNG VON (METH)ACRYLMONOMERE ENTHALTENDEN FLUIDEN**
METHOD FOR THE RECTIFICATIVE SEPARATION OF FLUIDS CONTAINING (METH)ACRYLIC MONOMERS
PROCÉDÉ POUR SÉPARER PAR RECTIFICATION DES FLUIDES CONTENANT DES MONOMÈRES (METH) ACRYLIQUES

(30) Priorität: 08.01.2003 DE 10300499
(43) Veröffentlichungstag der Anmeldung: 12.10.2005
(73) Patentinhaber: BASF SE, 67056 Ludwigshafen (DE)
(72) Erfinder: THIEL, Joachim, 67435 Neustadt (DE); DAMS, Albrecht, 67157 Wachenheim (DE); SCHRÖDER, Jürgen, 67071 Ludwigshafen (DE)
(86) Internationale Anmeldenummer: PCT/EP2003/014877
(87) Internationale Veröffentlichungsnummer: WO 2004/063137

(56) Entgegenhaltungen:
- EP-A- 1 044 957
- EP-A- 1 097 742
- DE-A- 10 115 277
- DATABASE WPI Section Ch, Week 200131 Derwent Publications Ltd., London, GB; Class A41, AN 2001-293790 XP002277806 & JP 2000 344688 A (MITSUBISHI CHEM CORP), 12. Dezember 2000 (2000-12-12) in der Anmeldung erwähnt

## Beschreibung

Vorliegende Erfindung betrifft ein Verfahren der rektifikativen Auftrennung von (Meth)acrylmonomere enthaltenden Fluiden in einer Rektifikationskolonne, bei dem zum Kopf der Rektifikationskolonne aufsteigender, (Meth)acrylmonomere enthaltender, Brüden unter Bildung von (Meth)acrylmonomere enthaltendem Kopfkondensat direkt gekühlt wird und wobei der Kondensationsraum am Kopf der Kolonne vom die trennwirksamen Einbauten enthaltenden Bereich der Rektifikationskolonne nur durch wenigstens einen Kaminboden abgetrennt ist, von dem gebildetes Kopfkondensat aus der Rektifikationskolonne entnommen wird.

Die Schreibweise "(Meth)acrylmonomere" steht in dieser Schrift verkürzend für "Acrylmonomere und/oder Methacrylmonomere".

Der Begriff "Acrylmonomere" steht in dieser Schrift verkürzend für "Acrolein, Acrylsäure und/oder Ester der Acrylsäure".

Der Begriff "Methacrylmonomere" steht in dieser Schrift verkürzend für "Methacrolein, Methacrylsäure und/oder Ester der Methacrylsäure".

Im besonderen sollen die in dieser Schrift angesprochenen (Meth)acrylmonomeren die nachfolgenden (Meth)acrylsäureester umfassen: Hydroxyethylacrylat, Hydroxyethylmethacrylat, Hydroxypropylacrylat, Hydroxypropylmethacrylat, Glycidylacrylat, Glycidylmethacrylat, Methylacrylat, Methylmethacrylat, n-Butylacrylat, iso-Butylacrylat, isoButylmethacrylat, n-Butylmethacrylat, tert.-Butylacrylat, tert.-Butylmethacrylat, Ethylacrylat, Ethylmethacrylat, 2-Ethylhexylacrylat, 2-Ethylhexylmethacrylat, N,N-Dimethylaminoethylacrylat und N,N-Dimethylaminoethylmethacrylat.

(Meth)acrylmonomere sind wichtige Ausgangsverbindungen zur Herstellung von Polymerisaten, die z.B. als Klebstoffe Verwendung finden.

(Meth)acrolein und (Meth)acrylsäure wird großtechnisch überwiegend durch katalytische Gasphasenoxidation geeigneter C₃-/C₄-Vorläuferverbindungen (oder von Vorläuferverbindungen dieser Vorläuferverbindungen), insbesondere von Propen und Propan im Fall von Acrolein und Acrylsäure bzw. von iso-Buten und iso-Butan im Fall der Methacrylsäure und des Methacroleins, hergestellt. Neben Propen, Propan, iso-Buten und iso-Butan eignen sich als Ausgangsstoffe jedoch auch andere 3 bzw. 4 Kohlenstoffatome enthaltende Verbindungen, beispielsweise iso-Butanol, n-Propanol oder der Ethylether von iso-Butanol (als C₄-Vorläufer). (Meth)acrylsäure kann auch aus (Meth)acrolein erzeugt werden.

Dabei wird normalerweise ein Produktgasgemisch erhalten, aus dem die (Meth)acrylsäure bzw. das (Meth)acrolein abgetrennt werden muß.

Diese Abtrennung wird in der Regel so durchgeführt, dass die (Meth)acrylsäure bzw. das (Meth)acrolein durch Absorption in ein Lösungsmittel (z.B. Wasser oder ein organisches Lösungsmittel) oder durch fraktionierende Kondensation des Produktgasgemisches zunächst grundabgetrennt und das dabei anfallende Kondensat bzw. Absorbat nachfolgend rektifikativ (in der Regel in mehreren Stufen) unter Erhalt von mehr oder weniger reiner (Meth)acrylsäure bzw. (Meth)acrolein aufgetrennt wird (vgl. z.B. EP-A 717029, EP-A 1125912, EP-A 982289, EP-A 982287, DE-A 19606877, DE-A 10115277, DE-A 10224341 und DE-A 10218419). Die vorstehend angesprochene fraktionierende Kondensation soll in dieser Schrift auch als unter den Begriff der Rektifikation fallend betrachtet werden. Sie unterscheidet sich von der herkömmlichen Rektifikation lediglich dadurch, dass das aufzutrennende Gemisch der Trennkolonne (der Rektifikationskolonne) gasförmig (d.h. vollständig in die Dampfform überführt) zugeführt wird. Der in dieser Schrift verwendete Begriff Fluide soll deshalb sowohl Flüssigkeiten als auch Gasgemische umfassen.

Ester der (Meth)acrylsäure sind z.B. durch direkte Umsetzung von (Meth)acrylsäure und/oder (Meth)acrolein mit den entsprechenden Alkoholen erhältlich. Allerdings fallen auch in diesem Fall zunächst Produktgasgemische an, aus denen die (Meth)acrylsäureester z.B. rektifikativ abgetrennt werden müssen.

Die vorstehend angesprochenen, (Meth)acrylmonomere enthaltenden und rektifikativ zu bearbeitenden Fluide bzw. Flüssigkeiten können die (Meth)acrylmonomere sowohl in mehr oder weniger reiner Form als auch in Lösung befindlich enthalten.

Das Lösungsmittel kann dabei sowohl wässrig als auch ein organisches Lösungsmittel sein. Die spezifische Art des Lösungsmittels ist erfindungsgemäß im wesentlichen unbeachtlich. Der Gehalt an (Meth)acrylmonomeren kann ≥ 2 Gew.-%, oder ≥ 5 Gew.-%, oder ≥ 10 Gew.-%, oder ≥ 20 Gew.-%, oder ≥ 40 Gew.-%, oder ≥ 60 Gew.-%, oder ≥ 80 Gew.-%, oder ≥ 90 Gew.-%, oder ≥ 95 Gew.-%, oder ≥ 99 Gew.-% betragen.

Die rektifikative Auftrennung der beschriebenen (Meth)acrylmonomere enthaltenden Fluide bzw. Flüssigkeiten kann je nach ihrer Zusammensetzung sowohl so erfolgen, dass sich die (Meth)acrylmonomere am Kopf der Rektifikationskolonne anreichern, als auch so, dass sich die (Meth)acrylmonomere im Sumpf der Rektifikationskolonne anreichern. Selbstredend können auch im oberen, unteren oder mittleren Teil der Rektifikationskolonne die (Meth)acrylmonomere angereichert enthaltenden Fraktionen entnommen werden.

In im wesentlichen allen vorgenannten Fällen besteht die Notwendigkeit, zum Kopf der Rektifikationskolonne aufsteigenden, mehr oder weniger (Meth)acrylmonomere enthaltenden, Brüden unter Bildung von (Meth)acrylmonomere enthaltendem Kopfkondensat zu kühlen. Vorstehendes gilt insbesondere dann, wenn das rektifikative Trennverfahren so betrieben wird, dass sich die (Meth)acrylmonomere am Kopf der Rektifikationskolonne anreichern.

Dies ist insofern problembehaftet, als (Meth)acrylmonomere in kondensierter Phase befindlich eine erhöhte Neigung zu unerwünschter Polymerisation aufweisen. Infolge dieses Sachverhalts ist das Auftreten von an Polymerisationsinhibitor freiem, (Meth)acrylmonomere enthaltendem, Kondensat in Rektifikationskolonnen zu vermeiden.

So empfiehlt z.B. die DE-A 2027655 für dieses Problemfeld die stete Benetzung kritischer Bauteile der Rektifikationskolonne mit polymerisationsinhibiertem Rücklauf. Die EP-A 1044957 beschreibt in entsprechender Weise spezifische Düsen, mittels derer Bauteile einer Rektifikationskolonne mit Polymerisationsinhibitor enthaltender Lösung besprüht werden. Die DE-A 2246480 empfiehlt dagegen als Problemlösung neuralgische Kolonnenbauteile zu überhitzen, um auf selbigen die Bildung von nicht inhibiertem Kondensat zu vermeiden (da Polymerisationsinhibitoren in der Regel nicht leicht flüchtig sind, ist die Dampfphase innerhalb einer Rektifikationskolonne normalerweise weitgehend frei an Polymerisationsinhibitor; Kondensatbildung auf Kolonnenbauteilen aus solcher Dampfphase heraus führt somit in natürlicher Weise zu an Polymerisationsinhibitor freiem Kondensat, dessen (Meth)acrylmonomere eine erhöhte Polymerisationsneigung aufweisen).

Nachteilig am Lösungsvorschlag der DE-A 2246480 ist, dass er zum Zweck der Brüdenkühlung nicht anwendbar ist.

Die EP-A 1034824 empfiehlt die relevante Brüdenkühlung außerhalb der Rektifikationskolonne mittels eines Rohrbündelwärmetauschers durchzuführen (externe Brüdenkühlung).

Nachteilig an dieser Lösungsvariante ist, dass sie einer gasseitigen Verbindung vom Kolonnenkopf zum Wärmetauscher bedarf. Ein solches Brüdenrohr weist in der Regel eine erhebliche Länge auf, da der Wärmetauscher aus statischen Gründen normalerweise auf Grund gelagert ist. Dies bedingt das Erfordernis, eine unerwünschte Polymerisation auch im Brüdenrohr zuverlässig zu unterdrücken. Zweckmäßig wird dazu das Brüdenrohr begleitbeheizt, um im Brüdenrohr eine Kondensatbildung weitestgehend auszuschließen. Ein weiterer Nachteil der Variante gemäß der EP-A 1034824 erwächst dann, wenn der zum Kopf der Rektifikationskolonne aufsteigende Brüden neben (Meth)acrylmonomeren noch Bestandteile enthält, die nicht oder nur bei extrem tiefen Temperaturen kondensieren und den Kondensationsbereich als Abgas verlassen.

Dies ist z.B. dann der Fall, wenn die Rektifikationskolonne mittels eines molekularen Sauerstoff enthaltenden Gases durchströmt wird (z.B. mit Luft), um die polymerisationsinhibierende Wirkung von molekularem Sauerstoff zu nützen. Es ist aber auch dann der Fall, wenn in der Rektifikationskolonne z.B. eine fraktionierende Kondensation von Produktgas einer heterogen katalysierten Gasphasenpartialoxidation von C₃-/C₄-Verbindungen durchgeführt wird (z.B. gemäß der DE-A 19924532 oder gemäß der DE-A 10247240). In diesen Fällen ist es erforderlich, den Anteil an kondensierbaren Bestandteilen im Abgas möglichst niedrig zu halten (z.B. aus Gründen einer erhöhten Ausbeute oder aus Umweltgründen). Eine scharfe Trennung von Abgas und kondensierbaren Bestandteilen bedarf einer besonders niederen Betriebstemperatur, falls sie mittels eines indirekten Wärmetauschers (z.B. eines Rohrbündelwärmetauschers) durchgeführt wird.

Die EP-A 1097742 empfiehlt ebenfalls eine externe Kühlung der relevanten Brüden. Sie bietet dabei sowohl eine indirekte Kühlung (z.B. mittels eines Rohrbündelwärmetauschers) als auch eine direkte Kühlung (z.B. durch Eindüsen von unterkühltem Kondensat) in einer von der Rektifikationskolonne räumlich getrennten Kühlvorrichtung an. Zum Zweck einer Trennung von Abgas und zu kondensierenden Bestandteilen (z.B. den (Meth)acrylmonomeren) empfiehlt die EP-A 1097742 die Nachschaltung eines separaten Nachkühlers. Nachteilig an der Empfehlung der EP-A 1097742 ist ebenfalls die Notwendigkeit eines verbindenden Brüdenrohres und der Bedarf eines zusätzlichen Apparates in Form des Nachkühlers.

Die JP-A 2000/344688 lehrt, die relevante Brüdenkühlung mittels eines in den Kopf der Rektifikationskolonne integrierten indirekten Wärmetauschers durchzuführen. Dadurch entfällt zwar die Notwendigkeit eines Brüdenrohres, nachteilig an dieser Verfahrensweise ist jedoch, dass die Wärmetauscheroberfläche kontinuierlich mit Polymerisationsinhibitor enthaltendem Kondensat besprüht werden muß. Außerdem erfordert auch hier die Indirektheit der Kühlung für eine scharfe Abgastrennung besonders niedere Betriebstemperaturen, was energetisch ungünstig ist.

Die DE-A 10220494 und die DE-A 10200583 offenbaren u.a., die relevante Brüdenkühlung auf dem Weg der direkten Kühlung in den Kopf der Rektifikationskolonne integriert vorzunehmen. Dabei wird dem Kopfbereich gemäß der DE-A 10200583 vorab gebildetes, unterkühltes und mit Polymerisationsinhibitor versetztes Kondensat zugeführt. Nachteilig an der Lehre der DE-A 10220494 ist, dass nähere Angaben zur konktreten Ausführung einer solchen direkten Kühlung fehlen.

Gemäß Ausführungsbeispiel der DE-A 10200583 erfolgt die in den Kolonnenkopf integrierte Brüdenkondensation mittels zweier hintereinandergeschalteter Direktkühlkreise (Quenchkreise). Der erste wird mittels unterkühltem, zuvor kondensiertem und mit Polymerisationsinhibitor versetztem Kopfkondensat (unterkühlt meint in dieser Schrift im gegebenen Zusammenhang stets, dass das Kopfkondensat nach seiner Entnahme aus der Rektifikationskolonne auf eine unterhalb der Entnahmetemperatur liegende Temperatur gebracht wird, bevor man es zum Zweck der Direktkühlung in die Rektifikationskolonne rückführt) und der zweite mittels gekühltem Wasser betrieben. Nachteilig an der Verfahrensweise der DE-A 10200583 ist, dass sie zwei voneinander verschiedene Kühlmittel anwendet und das bei der wässrigen Direktkühlung anfallende wässrige Kondensat einschließlich der darin enthaltenen Wertkomponenten der Entsorgung (z.B. in einer Kläranlage) zuführt. Für die DE-A 10256147 gilt das bezüglich der DE-A 10220494 Gesagte.

Die Aufgabe der vorliegenden Erfindung besteht daher darin, ein verbessertes Verfahren der rektifikativen Auftrennung von (Meth)acrylmonomere enthaltenden Fluiden in einer Rektifikationskolonne, bei dem zum Kopf der Rektifikationskolonne aufsteigender, (Meth)acrylmonomere enthaltender, Brüden unter Bildung von (Meth)acrylmonomere enthaltendem Kopfkondensat direkt gekühlt wird und wobei der Kondensationsraum am Kopf der Kolonne vom die trennwirksamen Einbauten enthaltenden Bereich der Rektifikationskolonne nur durch wenigstens einen Kaminboden abgetrennt ist, von dem gebildetes Kopfkondensat aus der Rektifikationskolonne entnommen wird, zur Verfügung zu stellen.

Demgemäß wurde ein Verfahren der rektifikativen Auftrennung von (Meth)acrylmonomere enthaltenden Fluiden in einer Rektifikationskolonne, bei dem zum Kopf der Rektifikationskolonne aufsteigender, (Meth)acrylmonomere enthaltender, Brüden unter Bildung von (Meth)acrylmonomere enthaltendem Kopfkondensat direkt gekühlt wird und wobei der Kondensationsraum am Kopf der Kolonne vom die trennwirksamen Einbauten enthaltenden Bereich der Rektifikationskolonne nur durch wenigstens einen Kaminboden abgetrennt ist, von dem gebildetes Kopfkondensat aus der Rektifikationskolonne entnommen wird, gefunden, das dadurch gekennzeichnet ist, dass die direkte Kühlung des Brüden im Kondensationsraum in wenigstens zwei, vom Brüden durchströmten, räumlich aufeinanderfolgenden Sprühzonen durch Versprühen von unterkühltem, Polymerisationsinhibitor zugesetzt enthaltendem, Kopfkondensat erfolgt, wobei die Temperatur des versprühten unterkühlten Kopfkondensats in Strömungsrichtung des Brüden von Sprühzone zu Sprühzone kleiner wird.

Der Vorteil des erfindungsgemäßen Verfahrens beruht darauf, dass zum einen kein Brüdenrohr erforderlich ist, das Kondensat automatisch polymerisationsinhibiert anfällt und zum anderen die Trennung von Abgas in energetisch vergleichsweise günstiger Weise scharf durchführbar ist.

Für den trennwirksame Einbauten enthaltenden Abschnitt der erfindungsgemäß zu verwendenden Rektifikationskolonne kommen alle gängigen Typen in Betracht. D.h., die Rektifikationskolonne kann z.B. eine Boden-, Füllkörper-, oder Packungskolonne sein. Selbstredend können vorgenannte Einbauten auch in gemischter Form angewendet werden.

Als Füllkörperschüttungen können z.B. Ringe, Wendeln, Sattelkörper, Raschig-, Intos- oder Pallringe, Barrell- oder Intalox-Sättel, Top-Pak oder Geflechte verwendet werden.

Als trennwirksame Böden kommen z.B. Glockenböden, Siebböden, Ventilböden, Tunnelböden, Thormannböden und/oder Dual-Flow-Böden in Betracht. Insbesondere ist das erfindungsgemäße Verfahren anwendbar bei Rektifikationskolonnen, die als trennwirksame Einbauten ausschließlich Dual-Flow-Böden enthalten (vgl. z.B. DE-A 10156988, EP-A 1029573 und DE-A 10230219).

Es ist aber auch anwendbar bei Rektifikationskolonnen und -verfahren, deren trennwirksamer Teil gemäß der DE-A 10243625 oder gemäß der DE-A 10247240 gestaltet und dessen Verfahren wie in diesen Schriften beschrieben durchgeführt wird.

Erfindungsgemäß wesentlich ist, dass der Kondensationsraum beim erfindungsgemäßen Verfahren unmittelbar auf dem die trennwirksamen Einbauten enthaltenden Teil der Rektifikationskolonne aufsitzt, und von diesem nur durch wenigstens einen Kaminboden getrennt ist. Erfindungsgemäß bevorzugt sind beide Räume nur durch einen Kaminboden voneinander getrennt.

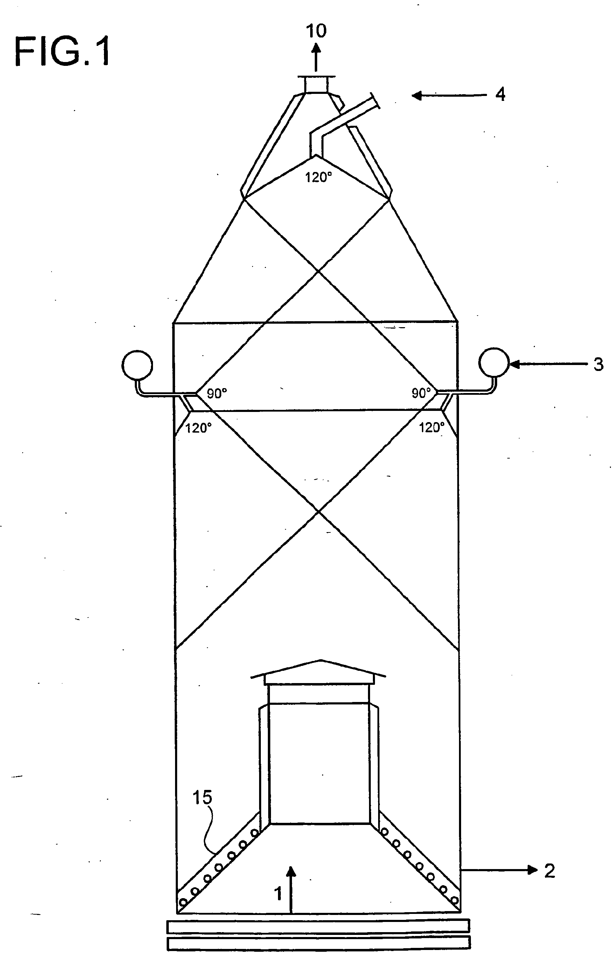

Kaminseitig führt der wenigstens eine Kaminboden in den Kondensationsraum. Dieser ist in seiner einfachsten Ausführungsform ein Leerrohr (d.h., ein an Einbauten im wesentlichen freier Raum), das zonenweise mit Sprühdosen versehen ist. Der Durchmesser des Leerrohres kann größer oder kleiner als der des Kaminbodens sein. Bevorzugt hat es an der Verbindungsstelle denselben Durchmesser wie der Kaminboden. Besonders bevorzugt verengt (verjüngt) sich das Leerrohr im oberen Abschnitt konisch auf den Durchmesser der Abgasableitung (vgl. Fig. 1). Großtechnisch kann der Größtdurchmesser des Leerrohres 0,5 bis 5 m, häufig 1 bis 3 m betragen. Die Höhe des Leerrohres beträgt großtechnisch typisch 1 bis 6 m, häufig 2 bis 4 m. Die Sprühdüsen einer Sprühzone sind normalerweise ringförmig im Leerrohr angebracht und werden bevorzugt über eine gemeinsame Ringleitung mit der zu versprühenden Flüssigkeit versorgt. Die Anzahl der Sprühdüsen pro Ringleitung beträgt großtechnisch in typischer Weise 5 bis 50, häufig 10 bis 30.

Erfindungsgemäß bevorzugt überlappen die Sprühkegel einer Sprühzone horizontal und vertikal (vgl. Fig. 1), so dass das gesamte Volumen homogen mit Sprühtröpfchen beaufschlagt werden kann.

Erfindungsgemäß sind wenigstens zwei übereinander angeordnete Ringleitungen mit Sprühdüsen erforderlich, wobei die Temperatur der zu versprühenden Flüssigkeit innerhalb der Ringleitungen von unten nach oben abnimmt. In diesem Fall ist es vorteilhaft, wenn sich die Sprühkegel der unterschiedlichen aufeinanderfolgenden Temperaturbereiche nur berühren und nicht überlappen. Letzteres bedingt eine erhöhte Kondensationseffizienz und eine schärfere Trennung von Abgas (10). Eine Überlappung wäre demgegenüber von Nachteil. In einer besonderen Ausführungsform des erfindungsgemäßen Verfahrens befindet sich eine letzte Sprühdüse unmittelbar unterhalb der Abgasleitung (vgl. Fig. 1), sofern Bedarf für eine solche Abgasleitung besteht.

Als Zerstäuberdüsen können erfindungsgemäß z.B. solche eingesetzt werden, wie sie die DE-A 19924533 beschreibt. Derartigen Düsen kann die zu versprühende Flüssigkeit unter Druck zugeführt werden. Die Zerteilung der zu versprühenden Füssigkeit kann dabei dadurch erfolgen, dass sie nach Erreichen einer bestimmten Mindestgeschwindigkeit in der Düsenbohrung entspannt wird. Ferner können für den erfindungsgemäßen Zweck auch Einstoffdüsen wie z.B. Drallkammern (Vollkegeldüsen) verwendet werden (z.B. von der Fa. Düsen-Schlick GmbH, DE, oder von der Sprayring Systems Deutschland GmbH).

Erfindungsgemäß bevorzugt sind die Sprühdüsen Vollkegelsprühdüsen mit einem Öffnungswinkel des Sprühkegels im Bereich von 60° bis 180°, bevorzugt 90° bis 120°. Der sich beim Versprühen einstellende Tröpfchendurchmesser ist erfindungsgemäß zweckmäßig s 1000 µm. Der Durchsatz je Sprühdüse beträgt in den weiter unten gelegenen Sprühzonen großtechnisch typisch 1 bis 50 m³/h, häufig 5 bis 20 m³/h. In den weiter oben gelegenen Sprühzonen beträgt diese Menge zweckmäßig 0,1 bis 10 m³/h, häufig 0,5 bis 5 m³/h. Die mittlere Fluglänge der Tröpfchen (bis sie an die Wand stossen) beträgt in den unteren Sprühzonen großtechnisch typisch 0,5 bis 10 m, häufig 1 bis 5 m. In den weiter oben gelegenen Sprühzonen liegen die mittleren Fluglängen großtechnisch typisch bei 0,05 bis1 m, häufig bei 0,1 bis 0,5 m.

Die Oberfläche des Kondensationsraums oberhalb des Sprühbereichs der letzten Sprühzone und die gegebenenfalls bestehende Abgasleitung können erfindungsgemäß zweckmäßig begleitbeheizt werden (5). Dabei sollte die Wandtemperatur 5 bis 10°C oberhalb der Temperatur liegen, die das Abgas besitzt, wenn es aus der letzten Sprühzone austritt.

Anwendungstechnisch zweckmäßig werden meist 2 bis 3 Sprühzonen angewendet.

Das durch die direkte Kühlung anfallende Kondensat wird auf dem Kaminboden gesammelt und von diesem z.B. über einen Stutzen mittels einer Pumpe abgezogen.

Das dem Kaminboden entnommene Kondensat (2) wird teilweise als Rücklauf (6) in den trennwirksame Einbauten enthaltenden Abschnitt der Rektifikationskolonne rückgeführt, teilweise als Zulauf der Sprühdüsen (nach Durchlaufen von indirekten Wärmetauschern (7), (8) zum Zweck der Unterkühlung und Zusatz von Polymerisationsinhibitor (zweckmäßig im Kondensat gelöst (9))) verwendet und teilweise als Produkt oder Nebenprodukt ausgeschleust (12).

Der Durchmesser des Kaminbodens entspricht anwendungstechnisch zweckmäßig dem Durchmesser des trennwirksamen Abschnitts der Rektifikationskolonne. Er kann aber auch 30 % bis 120 %, oder 50 % bis 100 % desselben betragen.

Großtechnisch typische Kaminbodendurchmesser betragen 0,5 bis 5 m, häufig 1 bis 3 m.

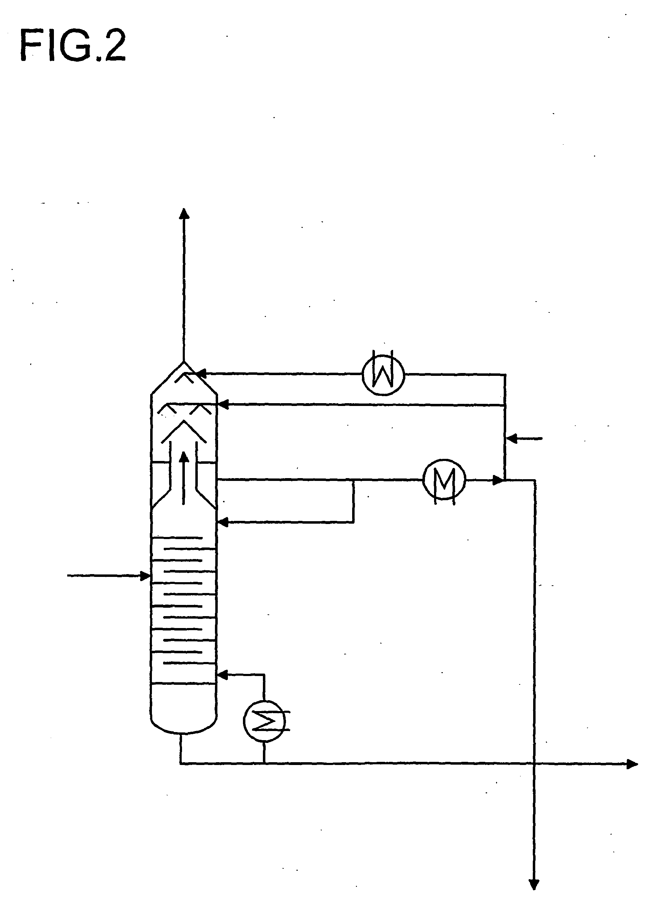

Bei einem Kaminboden mit nur einem Kamin ist dieser in der Regel mittig auf dem Kaminboden angebracht. Darüber hinaus hat er zweckmäßig ein (allseitiges) Gefälle vom Kamin zur Leerrohr(Kolonnen)innenwand (dies minimiert die auf dem Kaminboden auflaufende Kondensatmenge und verringert die Verweilzeit auf dem Boden). Der dadurch ausgebildete Ringspalt ist gleichzeitig die Pumpenvorlage (vgl. Fig. 1 und Fig. 2).

Weist der Kaminboden mehrere Kamine auf, ist eine Kaminanordnung und ein Gefälle des Kaminbodens wie in der DE-A 10159825 beschrieben vorteilhaft. Eine Abdeckhaube auf dem jeweiligen Kamin verhindert, dass Kondensat durch den Kamin in den darunter liegenden, die trennwirksamen Einbauten enthaltenden, Abschnitt der Rektifikationskolonne gelangt. Erfindungsgemäß bevorzugt handelt es sich dabei um Abdeckhauben, wie sie in der DE-A 10159825 beschrieben werden.

Der Durchmesser eines Kamins beträgt in der Regel ≥ 10 % und 5 70 % des Kaminbodendurchmessers. Häufig liegt der vorgenannte Prozentsatz im Bereich ≥ 25 % und s 50 %. Typische Durchmesserwerte bei großtechnischer Anwendung betragen 0,2 bis 1,5 m, häufig 0,5 bis 1,0 m, bei Kaminhöhen von 1 bis 3 m, bzw. 1,5 bis 2,5 m.

Erfindungsgemäß bevorzugt sind sowohl die Kamine als auch der Kaminboden gegen den trennwirksamen Abschnitt der Rektifikationskolonne thermisch isoliert ausgeführt, um auf der dem trennwirksamen Abschnitt zugewandten Seite unkontrollierte Kondenstation zu vermeiden bzw. zu mindern. In einfachster Weise sind sie zu diesem Zweck doppelwandig konzipiert. Erfindungsgemäß bevorzugt wird dabei auf der oberen Fläche der unteren der beiden Wände des Kaminbodens (Ringspalts) eine Begleitheizung (z.B. von einem Wärmeträger durchströmte Rohre bzw. Schläuche ((15) in Fig. 1) aufgebracht.

Bevorzugt wird diese Begleitheizung erst nach Inbetriebnahme der Rektifikation eingeschaltet.

Wie bereits erwähnt, wird der Zulauf zu den Sprühdüsen mittels indirekter Wärmetauscher unterkühlt. Dazu ist der Einsatz mehrerer in Reihe oder parallel geschalteter Wärmetauscher möglich. Üblicherweise wird um 5 bis 25°C unterhalb der Entnahmetemperatur vom Kaminboden unterkühlt. Besteht das Kondensat zu mehr als 90 Gew.-% aus (Meth)acrylmonomeren, wird als Kühlmittel in den Wärmetauschern wenigstens teilweise z.B. Flusswasser verwendet. Dabei ist es zweckmäßig, einen Sekundärkühlkreis zwischenzuschalten. Dieser verhindert den Produktaustritt in das Kühlwasser bei Undichtigkeiten der Wärmetauscher.

Bei einer rektifikativen über Kopf Abtrennung von Acrylsäure oder Methacrylsäure mit einem Säuregehalt ≥ 90 Gew.-% im Kondensat und alleiniger Polymerisationsinhibierung mittels Hydrochinonmonomethylether (MEHQ) sowie gegebenenfalls durchströmender Luft sind Ablauftemperaturen des Kondensats von ≥ 40°C zu vermeiden. Der Gehalt an MEHQ im Kondensat beträgt zweckmäßig 50 bis 250 Gew.ppm.

Erfindungsgemäß vorteilhaft wird die insgesamt verwendete Menge an Sprühkondensat nach seiner Entnahme vom Kaminboden zunächst über einen ersten indirekten (vorzugsweise mit Flusswasser betriebenen) Wärmetauscher geführt. Davon wird eine erste Teilmenge (3) der ersten Sprühzone zugeführt und die Restmenge zum Zweck der weiteren Kühlung einem zweiten indirekten (vorzugsweise mit Kühlsole betriebenen) Wärmetauscher zugeführt, bevor sie vollständig (4) oder teilweise an die zweite Sprühzone weitergeleitet wird etc. (vgl. Fig. 2).

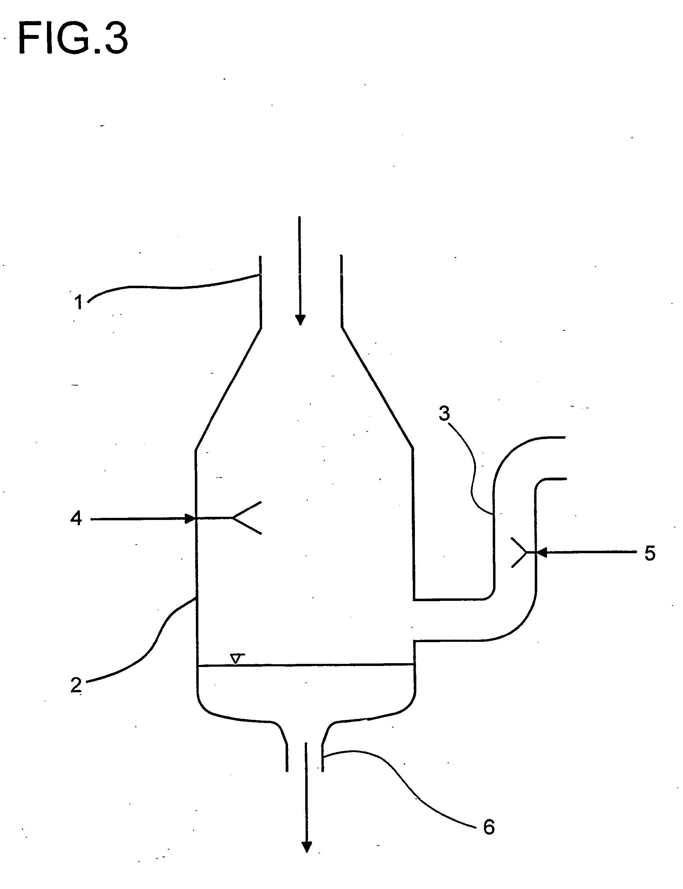

In einer alternativen Ausführungsform kann der Kondensationsraum auch wie in Figur 3 beschaffen sein.

Über ein Brüdenrohr (1) wird der zu kühlende Brüden in den Kondensationsraum geführt, der aus einem Hauptraum (2) und aus einem Nebenarm (3) besteht.

Sowohl im Hauptraum als auch im Nebenarm befindet sich eine Ringleitung mit Sprühdüsen ((4) bzw. (5)).

Die Temperatur T₂ des Zulaufs zu den Sprühdüsen (5) liegt tiefer als die Temperatur T₁ des Zulaufs zu den Sprühdüsen (4).

Das Kondensat wird über die Leitung (6) weggeführt. Davon wird eine Teilmenge als Produkt ausgelassen, eine Teilmenge als Rücklauf für die Rektifikationskolonne verwendet und eine Teilmenge nach Durchgang durch die entsprechenden indirekten Wärmetauscher in abgekühlter Form den Sprühdüsen (4) bzw. (5) zugeführt.

Das erfindungsgemäße Verfahren ist im Fall aller eingangs dieser Schrift genannten (Meth)acrylmonomere anwendbar.

Es ist insbesondere anwendbar im Fall einer rektifikativen Abtrennung von Acrylsäure aus Acrylsäure enthaltenden Gemischen, wie sie die EP-A 717029, die EP-A 839790, die US-A 5482597, die EP-A 713854, die DE-A 19634614, die US-A 5710329, die EP-A 1110940, die DE-A 19853064, die DE-A 4201697, die DE-A 3641996, die EP-A 1033359, die DE-A 10138150, die DE-A 10138101 und die EP-A 648732 beschreiben. Das gleiche gilt für die Verfahren der EP-A 648732, der EP-A 713854, der US-A 5482597, der EP-A 839790, der DE-A 1980962 und der DE-A 4335172.

Alle in dieser Schrift verwendeten numerischen Adressen beziehen sich auf die Figuren 1 und 2. Die (11) meint das aufzutrennende Gemisch, die (13) meint entnommene Sumpfflüssigkeit, die nach ihrer indirekten Erwärmung in den unteren Teil der Rektifikationskolonne rückgeführt wird und die (14) meint ausgeschleuste Schwersieder, die in der Regel einer thermischen Rückspaltung zugeführt werden.

Der Gehalt an (Meth)acrylmonomeren der erfindungsgemäß aufzutrennenden (Meth)acrylmonomere enthaltenden fluiden Gemische kann die eingangs dieser Schrift genannten Werte aufweisen.

Als Polymerisationsinhibitoren kommen alle im Stand der Technik bekannten in Betracht. Typische Vertreter solcher Polymerisationsinhibitoren sind Phenothiazin, 4-Methoxyphenol und 4-Hydroxy-2,2,6,6-tetramethylpiperidin-N-oxyl.

Die im einzelnen für die jeweilige Sprühzonen anzuwendenden Temperaturen kann der Fachmann für das jeweilige spezifische Trennproblem anhand weniger Versuche ermitteln. In der Regel wird man das zu versprühende Kopfkondensat beim erfindungsgemäßen Verfahren überinhibiert versprühen, so dass das resultierende neue Kopfkondensat in ausreichendem Umfang Polymerisationsinhibitor enthält.

Abschließend seien die Vorteile des erfindungsgemäßen Verfahrens nochmals zusammengefaßt. Das erfindungsgemäße Verfahren benötigt kein die Trenneinheit und die Kondensationseinheit verbindendes Brüdenrohr. Die im anfallenden Kondensat enthaltene Kondensationswärme kann in einem ersten indirekten Wärmetauscher in der Regel bei einer vergleichsweise hohen Temperatur abgeführt werden, so dass der Wärmetauscher zum Zweck der Unterkühlung des Kondensats meist mit Flusswasser betrieben werden kann (der Entnahmestrom 12 kann auch vorab des Eintritts des entnommenen Kondensats in den Wärmetauscher abgezweigt werden). Durch die Anwendung von hintereinandergeschalteten Sprühzonen erfolgt eine kondensative Trennung von Abgas scharf. Der dazu in den höher gelegenen Sprühzonen benötigte Durchsatz an zu versprühendem Kondensat ist vergleichsweise gering. Dies ist insofern von Vorteil, als das in ihnen versprühte Kondensat zum Erreichen der niederen Kühltemperaturen meist in mit Kühlsolen betriebenen Wärmetauschern einer zweiten Kühlung unterworfen wird. Der Solebedarf ist diesbezüglich vergleichsweise klein. Insgesamt ist die erfindungsgemäße Kondensationseinheit somit energetisch günstig zu betreiben.

Zur Durchführung des erfindungsgemäßen Verfahrens bedarf es lediglich einer Rektifikationskolonne, die einen trennwirksame Einbauten enthaltenden Abschnitt umfasst, der nach oben durch wenigstens einen Kaminboden abgeschlossen und in einen Sprühkondensator mit wenigstens zwei Sprühzonen fortgeführt wird. Der Sprühkondensator steht mit der entsprechenden Anzahl an Wärmetauschern in Verbindung.

## Patentansprüche

1. Verfahren der rektifikativen Auftrennung von (Meth)acrylmonomere enthaltenden Fluiden in einer Rektifikationskolonne, bei dem zum Kopf der Rektifikationskolonne aufsteigender, (Meth)acrylmonomere enthaltender, Brüden unter Bildung von (Meth)acrylmonomere enthaltendem Kopfkondensat direkt gekühlt wird und wobei der Kondensationsraum am Kopf der Kolonne vom die trennwirksamen Einbauten enthaltenden Bereich der Rektifikationskolonne nur durch wenigstens einen Kaminboden abgetrennt ist, von dem gebildetes Kopfkondensat aus der Rektifikationskolonne entnommen wird, **dadurch gekennzeichnet, dass** die direkte Kühlung des Brüden im Kondensationsraum in wenigstens zwei, vom Brüden durchströmten, räumlich aufeinanderfolgenden Sprühzonen durch Versprühen von unterkühltem, Polymerisationsinhibitor zugesetzt enthaltendem, Kopfkondensat erfolgt, wobei die Temperatur des versprühten unterkühlten Kopfkondensats in Strömungsrichtung des Brüden von Sprühzone zu Sprühzone kleiner wird.

2. Verfahren nach Anspruch 1, **dadurch gekennzeichnet, dass** eine Sprühzone über ringförmig angebrachte Sprühdüsen versorgt wird.

3. Verfahren nach Anspruch 2, **dadurch gekennzeichnet, dass** es sich bei den Sprühdüsen um Vollkegelsprühdüsen handelt, deren Öffnungswinkel 60° bis 180° beträgt.

4. Verfahren nach Anspruch 3, **dadurch gekennzeichnet, dass** der Öffnungswinkel 90° bis 120° beträgt.

5. Verfahren nach Anspruch 3 oder 4, **dadurch gekennzeichnet, dass** sich die Sprühkegel ein und derselben Sprühzone überlappen.

6. Verfahren nach einem der Ansprüche 3 bis 5, **dadurch gekennzeichnet, dass** sich die Sprühkegel von räumlich aufeinanderfolgenden Sprühzonen nicht überlappen.

7. Verfahren nach einem der Ansprüche 3 bis 6, **dadurch gekennzeichnet, dass** sich die Sprühkegel von räumlich aufeinanderfolgenden Sprühzonen gerade berühren.

8. Verfahren nach einem der Ansprüche 1 bis 7, **dadurch gekennzeichnet, dass** die Rektifikationskolonne von einem molekularen Sauerstoff enthaltenden Gas durchströmt wird.

9. Verfahren nach einem der Ansprüche 1 bis 8, **dadurch gekennzeichnet, dass** der Kondensationsraum eine Abgasableitung aufweist.

10. Verfahren nach Anspruch 9, **dadurch gekennzeichnet, dass** der Kondensationsraum ein Leerrohr ist, das sich zur Abgasableitung hin konisch verjüngt.

11. Verfahren nach einem der Ansprüche 1 bis 10, **dadurch gekennzeichnet, dass** der Kaminboden ein allseitiges Gefälle zur Innenwand der Kondensationsraumes aufweiset.

12. Verfahren nach einem der Ansprüche 1 bis 11, **dadurch gekennzeichnet, dass** Kamin und Kaminboden gegen den trennwirksame Einbauten enthaltenden Abschnitt der Rektifikationskolonne thermisch isoliert ausgeführt sind.

13. Verfahren nach einem der Ansprüche 1 bis 12, **dadurch gekennzeichnet, dass** Kamin und Kaminboden doppelwandig ausgeführt sind.

14. Verfahren nach Anspruch 13, **dadurch gekennzeichnet, dass** auf der oberen Fläche der unteren der beiden Wände eine Begleitheizung angebracht ist.

15. Rektifikationskolonne umfassend einen trennwirksame Einbauten enthaltenen Abschnitt, der nach oben durch wenigstens einen Kaminboden abgeschlossen und in einen Sprühkondensator mit wenigstens zwei Sprühzonen fortgeführt wird.

## Claims

1. A process for rectificatively separating fluids comprising (meth)acrylic monomers in a rectification column by directly cooling the vapor comprising (meth)acrylic monomers rising to the top of the rectification column to form top condensate comprising (meth)acrylic monomers, the condensation space at the top of the column being separated from the region of the rectification column comprising the separating internals only by at least one chimney tray from which the top condensate form is removed from the rectification column, which comprises effecting the direct cooling of the vapor in the condensation space in at least two spray zones, which are spatially successive and are flowed through by vapor, by spraying supercooled top condensate comprising added polymerization inhibitor, and the temperature of the sprayed supercooled top condensate becoming lower from spray zone to spray zone in the flow direction of the vapor.

2. The process according to claim 1, wherein a spray zone is supplied via annularly mounted spray nozzles.

3. The process according to claim 2, wherein the spray nozzles are full cone spray nozzles whose opening angle is from 60° to 180°.

4. The process according to claim 3, wherein the opening angle is from 90° to 120°.

5. The process according to claim 3 or 4, wherein the spray cones overlap one and the same spray zone.

6. The process according to any of claims 3 to 5, wherein the spray cones of spatially successive spray zones do not overlap.

7. The process according to any of claims 3 to 6, wherein the spray cones of spatially successive spray zones just touch.

8. The process according to any of claims 1 to 7, wherein the rectification column is flowed through by a molecular oxygen-comprising gas.

9. The process according to any of claims 1 to 8, wherein the condensation space has an offgas outlet.

10. The process according to claim 9, wherein the condensation space is an empty pipe which narrows conically toward the offgas outlet.

11. The process according to any of claims 1 to 10, wherein the chimney tray has a slope on all sides toward the inner wall of the condensation space.

12. The process according to any of claims 1 to 11, wherein chimney and chimney tray are configured with thermal isolation against the section of the rectification column comprising the separating internals.

13. The process according to any of claims 1 to 12, wherein chimney and chimney tray have a double-walled configuration.

14. The process according to claim 13, wherein trace heating is mounted to the upper surface of the inner of the two walls.

15. A rectification column comprising a section which comprises separating internals and is completed at the top by at least one chimney tray and is continued into a spray condenser having at least two spray zones.

## Revendications

1. Procédé de séparation par rectification de fluides contenant des monomères (méth)acryliques dans une colonne de rectification, dans lequel de la buée montant vers la tête de la colonne de rectification et contenant des monomères (méth)acryliques est refroidi directement avec formation d'un condensat de tête contenant des monomères (méth)acryliques et l'espace de condensation à la tête de la colonne n'est séparé du secteur de la colonne de rectification contenant les garnissages à action séparatrice que par au moins un plateau à cheminées, à partir duquel du condensat de tête formé est prélevé dans la colonne de rectification, **caractérisé en ce que** le refroidissement direct de la buée dans l'espace de condensation a lieu dans au moins deux zones de pulvérisation consécutives dans l'espace traversées par la buée, grâce à la pulvérisation d'un condensat de tête surfondu contenant un apport d'inhibiteur de polymérisation, la température du condensat de tête surfondu pulvérisé se réduisant de zone de pulvérisation en zone de pulvérisation dans le sens d'écoulement de la buée.

2. Procédé selon la revendication 1, **caractérisé en ce qu'**une zone de pulvérisation est alimentée par l'intermédiaire de buses de pulvérisation installées de manière annulaire.

3. Procédé selon la revendication 2, **caractérisé en ce qu'**il s'agit, en ce qui concerne les buses de pulvérisation, de buses de pulvérisation coniques, dont l'angle d'ouverture est de 60° à 180°.

4. Procédé selon la revendication 3, **caractérisé en ce que** l'angle d'ouverture est de 90° à 120°.

5. Procédé selon la revendication 3 ou 4, **caractérisé en ce que** les cônes de pulvérisation se chevauchent sur une seule et même zone de pulvérisation.

6. Procédé selon l'une quelconque des revendications 3 à 5, **caractérisé en ce que** les cônes de pulvérisation de zones de pulvérisation consécutives dans l'espace ne se chevauchent pas.

7. Procédé selon l'une quelconque des revendications 3 à 6, **caractérisé en ce que** les cônes de pulvérisation de zones de pulvérisation consécutives dans l'espace se rejoignent précisément.

8. Procédé selon l'une quelconque des revendications 1 à 7, **caractérisé en ce que** la colonne de rectification est traversée par un gaz contenant de l'oxygène moléculaire.

9. Procédé selon l'une quelconque des revendications 1 à 8, **caractérisé en ce que** l'espace de condensation présente une dérivation pour un effluent gazeux.

10. Procédé selon la revendication 9, **caractérisé en ce que** l'espace de condensation est un tube vide se rétrécissant coniquement vers la dérivation d'effluent gazeux.

11. Procédé selon l'une quelconque des revendications 1 à 10, **caractérisé en ce que** le plateau à cheminées présente de tous côtés une pente vers la paroi interne de l'espace de condensation.

12. Procédé selon l'une quelconque des revendications 1 à 11, **caractérisé en ce que** la cheminée et le plateau à cheminées sont réalisés de manière thermiquement isolée contre la section de la colonne de rectification qui contient le garnissage à action séparatrice.

13. Procédé selon l'une quelconque des revendications 1 à 12, **caractérisé en ce que** la cheminée et le plateau à cheminées sont dotés de double paroi.

14. Procédé selon la revendication 13, **caractérisé en ce qu'**un traçage est appliqué à la surface supérieure de la plus basse des deux parois.

15. Colonne de rectification comportant une section, contenant un garnissage à action séparatrice, qui se termine vers le haut par au moins un plateau à cheminées et se poursuit dans un appareil de grelonage comportant au moins deux zones de pulvérisation.
